# EUROPEAN PATENT APPLICATION

(11) **EP 4 744 696 A1**
(43) Date of publication of application: **20.05.2026**
(21) Application number: 24306906.9
(22) Date of filing: 14.11.2024
(51) Int. Cl.: A61M 5/00, A61M 5/28, A61M 5/31, A61M 5/32

(54) **REUSABLE ADD-ON CASING FOR AN INJECTION DEVICE**

(71) Applicant: Becton Dickinson France, 38800 Le Pont-de-Claix (FR)
(72) Inventor: BESSON, Nicolas, 38650 TREFFORT (FR)
(74) Representative: Germain Maureau

(57) **Abstract**

This casing (1) includes: a body (2) delimiting an inner cavity for receiving the injection device (5), the body (2) including a proximal end (204) and a distal end (202) configured for axially abutting against an injection site; a proximal stop (22) arranged on the body (2) for blocking distal movement of the injection device (5) at a predetermined axial position within the body (2); and a snap-fit member (3) arranged on the body (2) for blocking proximal movement of the injection device (5), the snap-fit member (3) being resiliently deformable between a release position allowing insertion or removal of the injection device (5), and a locked position in which the snap-fit member (3) prevents removal of the injection device (5) from the body (2).

## Description

The present invention relates to a reusable add-on casing for an injection device.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

In this application, inner or inward means oriented towards a central longitudinal axis A, and outer or outward means oriented opposite to the central longitudinal axis A.

It is known to use injection devices including a syringe having an injection needle and a barrel designed for containing a medical product, a body for receiving the syringe, a guard for covering the injection needle after injection, a plunger rod to dispense the medical product from the syringe and a spring for activation of the guard. The guard and the body are held together by two trigger fingers prior to use of the device. At the end of injection, the plunger rod abuts against the trigger fingers and deflect the trigger fingers such that the guard is released from the body. Under the action of the spring, the guard distally moves to its safety position.

When using this injection device, the user needs to prick a patient at an appropriate skin depth. Inserting the injection needle at an inappropriate needle exposure length may result in injecting the medical product in the wrong tissues, for instance in muscular tissues instead of subcutaneous tissues.

There is therefore a need for a device that permits to control the needle exposure length. There is also a need for a device that is ergonomic so that even untrained users would be able to perform injection properly. There is also a need for a device that is reusable to limit the environmental impact.

In this context, an aspect of the invention is a casing configured for receiving an injection device, the casing including:
a body delimiting an inner cavity for receiving the injection device, the body including a proximal end and a distal end configured for axially abutting against an injection site,
a proximal stop arranged on the body for blocking distal movement of the injection device at a predetermined axial position within the body,
a snap-fit member arranged on the body for blocking proximal movement of the injection device, the snap-fit member being resiliently deformable between a release position allowing insertion or removal of the injection device, and a locked position in which the snap-fit member prevents removal of the injection device from the body.

The casing of the invention thus permits to control the needle exposure length. Besides, thanks to the snap-fit member that can release the injection device, the casing can be re-used. The casing is an add-on device, capable of being removably added to an injection device in order to add or improve functions of the injection device. The used injection device can be withdrawn from the casing of the invention after moving the snap-fit member to the release position.

The casing of the invention may further include some or all of the features listed below.

In an embodiment, the body includes two diametrically opposite snap-fit members.

In an embodiment, the snap-fit member axially faces the proximal stop so that the injection device is axially blocked within the body between the snap-fit member and the proximal stop.

In an embodiment, the snap-fit member and the proximal stop are provided on a finger flange of the body.

In an embodiment, the snap-fit member and the body are made of a single piece.

In an embodiment, the snap-fit member includes an inward radial protrusion having an inclined distal side for abutting against the injection device.

In an embodiment, the release position of the snap-fit member is a deformed position.

In an embodiment, the snap-fit member is a proximally extending leg having a proximal end fixed to the body, a free distal end, a bent portion arranged between the proximal end and the free distal end, and a pushing surface defined between the bent portion and the free distal end for allowing a user to outwardly deform the snap-fit member from the locked position to the release position.

In an embodiment, the body includes two slots respectively extending on each side of the snap-fit member.

In an embodiment, the body includes one or more radial bumps configured for radially abutting against the injection device when the injection device is positioned within the body.

In an embodiment, the body includes a see-through window.

In one embodiment, the distal end of the body includes a ring which radially outwardly protrudes from the distal end of the body.

In an embodiment, the casing includes a cap removably attached to the body, the cap including a gripping member configured for gripping a needle shield of the injection device, such that removal of the cap from the body entails removal of the needle shield from the injection device, and a release element configured for releasing the needle shield.

According to another aspect, the invention relates to a drug delivery device including the above-described casing and an injection device arranged within the casing. The injection device may further be provided with a safety device. The safety device is configured for protecting the user against needle stick injuries after injection.

The invention and the advantages arising therefrom will clearly emerge from the detailed description that is given below with reference to the appended drawings as follows :
- Figure 1 is a cross-section view of a safety device of a drug delivery device according to an embodiment of the invention,
- Figure 2 is a perspective view of an injection device of a drug delivery device according to an embodiment of the invention,
- Figure 3 is a perspective view of a drug delivery device according to an embodiment of the invention,
- Figures 4A, 4B, 4C are, respectively, a side view, a cross-section view and a perspective view of a casing according to an embodiment of the invention,
- Figures 5A, 5B are perspective views of a body of a casing according to an embodiment of the invention,
- Figure 5C is a side view illustrating a snap-fit member of a casing according to an embodiment of the invention,
- Figures 6A, 6B are, respectively, a perspective view and a cross-section view of a casing according to an embodiment of the invention, when the injection device is positioned within the body,
- Figures 7A to 7E illustrates use of the casing according to an embodiment of the invention,
- Figure 8 is a perspective view of a drug delivery device according to another embodiment of the invention,
- Figure 9A is a perspective view of a cap of the casing according to an embodiment of the invention,
- Figures 9B and 9C are partial cross-section views illustrating the gripping member of the cap of Figure 9A in, respectively, the gripping position and the release position,
- Figure 9D is a cross-section view of the cap of Figure 9A,
- Figures 10A-10F are views of a cap of a casing according to another embodiment of the invention,
- Figures 11A-11C are views of a cap of a casing according to another embodiment of the invention,
- Figures 12A and 12B are views of a cap of a casing according to another embodiment of the invention,
- Figures 13A to 13G are views of a cap of a casing according to another embodiment of the invention.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

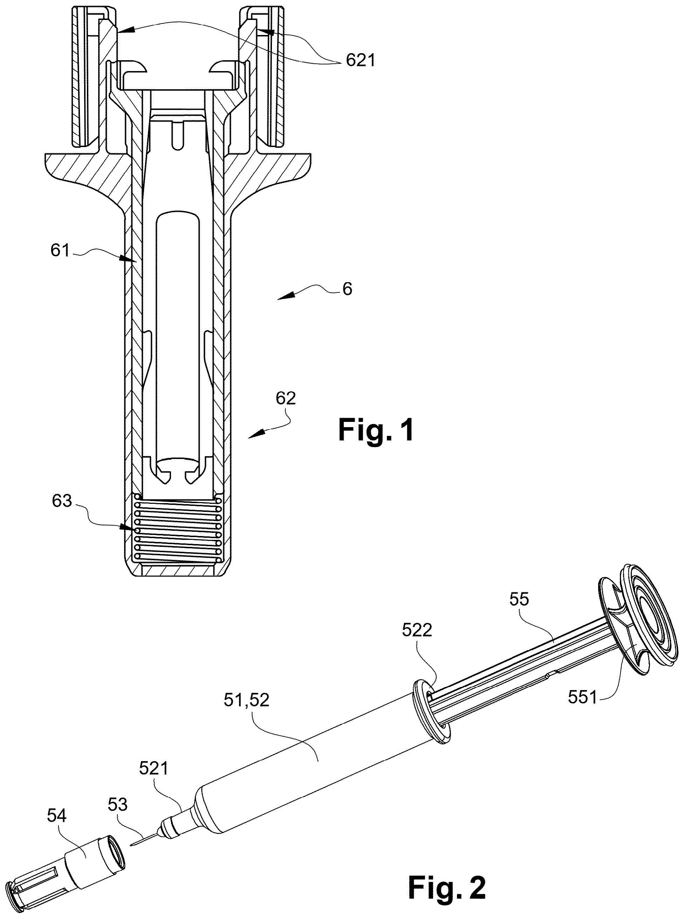

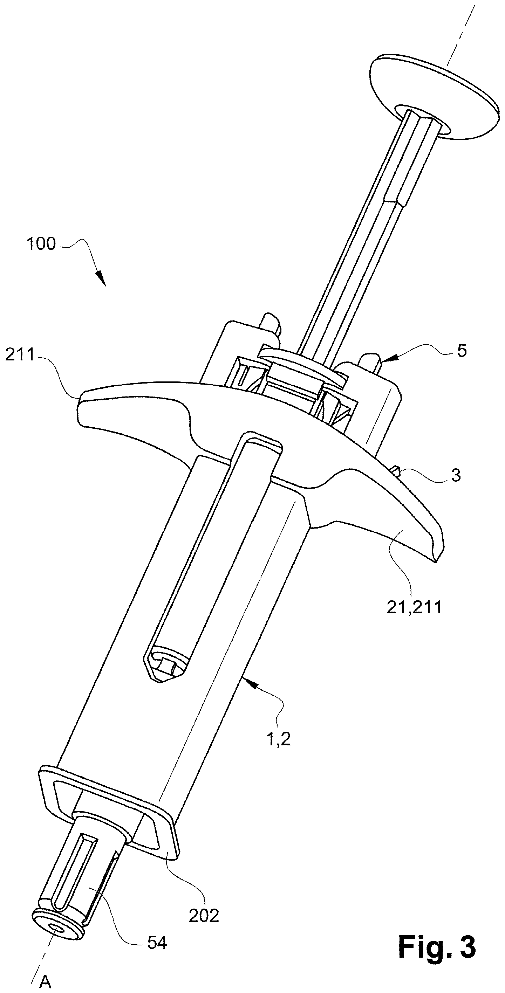

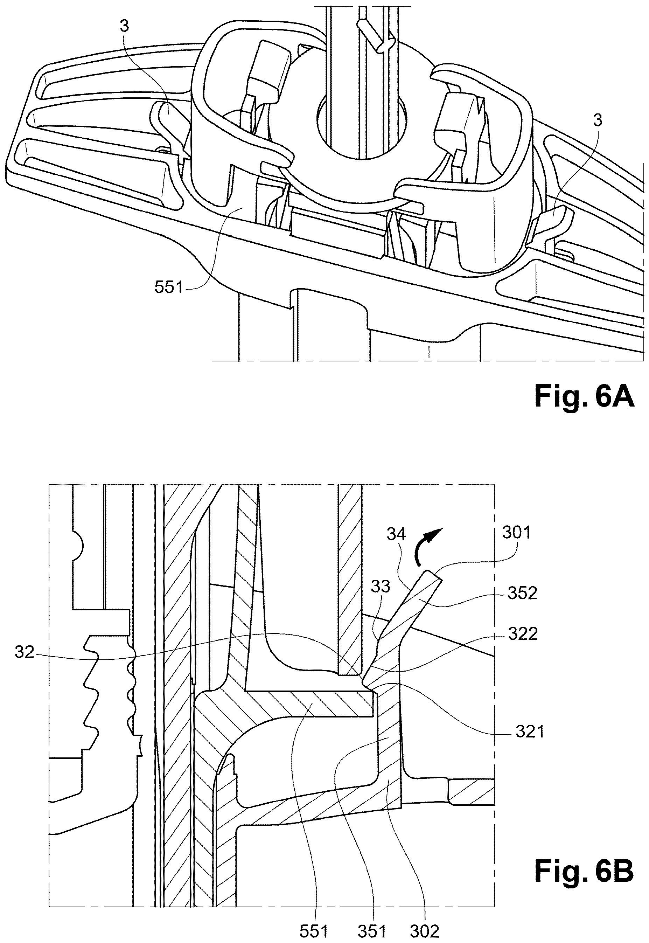

With reference to Figure 3 is shown a casing 1 according to an embodiment of the invention. The casing 1 is configured for housing an injection device 5. The injection device 5 may include a medical container 51, such as a prefillable or prefilled syringe. As shown in Figure 2, the medical container 51 includes a barrel 52 defining a reservoir for containing a medical product. The barrel 52 may include a longitudinal distal tip 521 to which is attached an injection needle 53 fluidly connected to the reservoir. A needle shield 54 is removably attached to the distal tip 521 to protect and seal the injection needle 53 before use. A plunger rod 55 is configured for extending through an opened proximal end 522 of the barrel 52 to push a stopper 56, Figure 6B, arranged within the barrel 52, thereby expelling the medical product outside the medical container 51 via the distal tip 521 and the injection needle 53.

The medical container 51, illustrated in Figure 2, may be contained within a safety device 6 of the type illustrating in Figure 1, including a body 61 for receiving the medical container 51, a guard 62 for covering the injection needle 53 after injection, and a spring 63 for activation of the guard. The guard and the body are held together by two trigger fingers 621 prior to use of the device. At the end of injection, the plunger rod 55 abuts against the trigger fingers 621 and deflect the trigger fingers 621 such that the guard 62 is released from the body 61 and can deploy in the distal direction, under the action of the spring 63, to cover the injection needle 53.

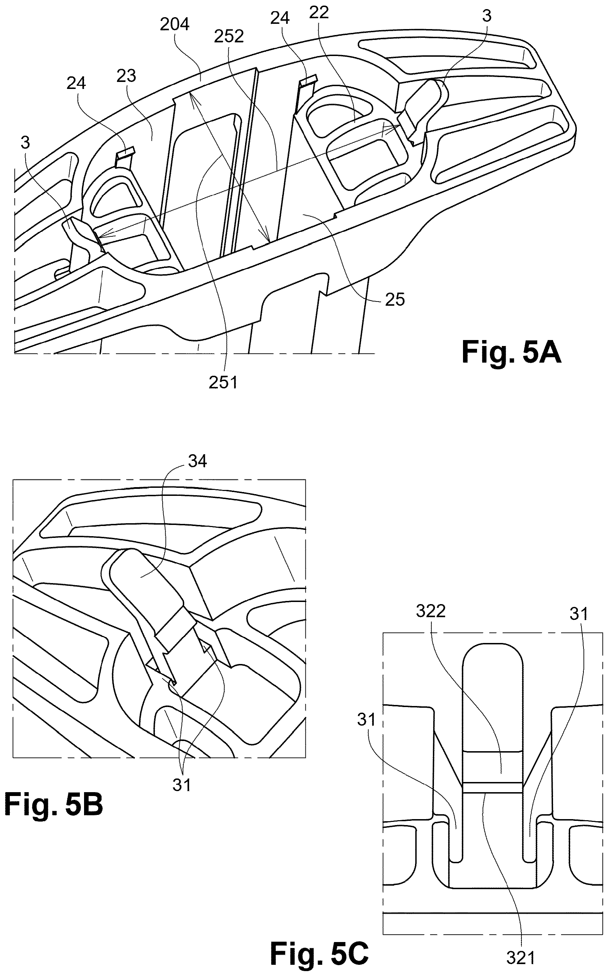

The casing 1 of the invention may include a body 2 delimiting an inner cavity for receiving the injection device 5, a proximal stop 22, Figure 5A, arranged on the body 2 for blocking distal movement of the injection device 5 at a predetermined axial position within the body 2, and a snap-fit member 3 arranged on the body 2 for blocking proximal movement of the injection device 5. The body 2 includes an opened proximal end 204 for insertion of the injection device 5, and an opposite distal end 202, Figure 3, for axially abutting against an injection site. The snap-fit member 3 is resiliently deformable between a release position allowing insertion or removal of the injection device, and a locked position in which the snap-fit member 3 prevents removal of the injection device 5 from the body 2.

The outer body 2 extends along a longitudinal axis A and around the injection device 5. The outer body 2 may in the form of a tubular sleeve, having a cylindrical, elliptic, parallelepipedic or polygonal shape allowing for ergonomic seizing of the casing 1 by the user. The outer body 2 has a lateral wall 20 defining an inner cavity for containing the injection device 5. The lateral wall 20 may include a see-through window 201 allowing for visual inspection of the medical container 51.

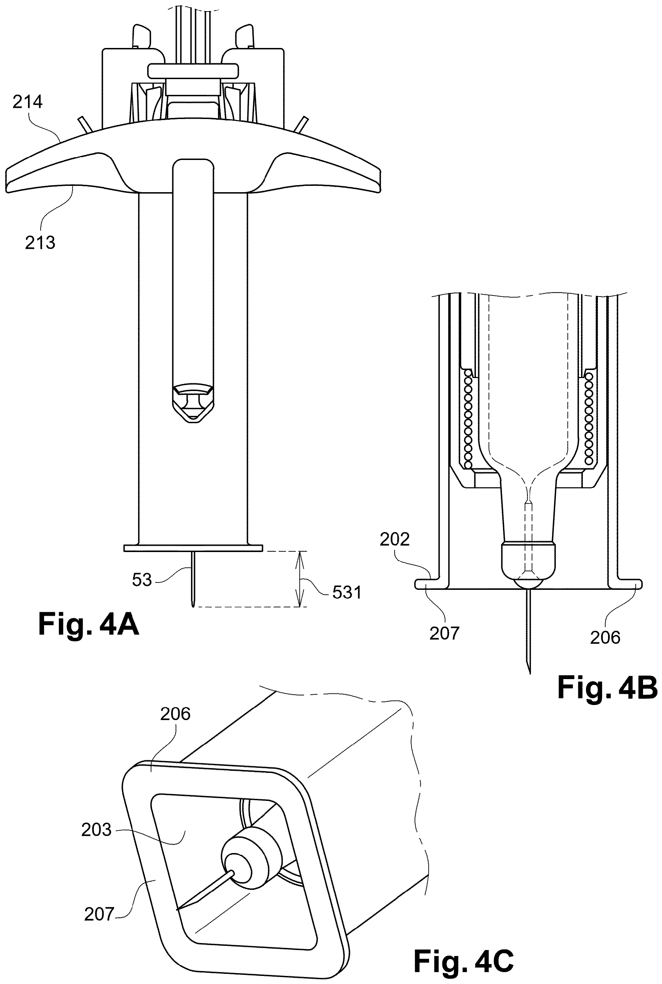

The distal end 202 of the body 2 delimits a distal opening 203, Figure 4C, configured for allowing extension of the guard 62 of the injection device 5 after injection. The outer body 2 has a finger flange 21, Figure 3, which may radially outwardly protrude at the opened proximal end 204 of the outer body 2, for providing ergonomic support to the user's fingers. The finger flange 21 may be defined by two diametricallly opposite radial extensions 211. In one embodiment, Figure 4A, the finger flange 21 may have a convex proximal side 213, and/or a concave distal side 214. In other embodiments (not shown), the finger flange 21 may have a circular or oval shape, or may have a square, rectangular, or any other polygonal shape. The finger flange 21 may thus extend all around the proximal end 204 of the outer body 2, and not solely at the two radial extensions 211. The finger flange 21 is designed to avoid hindrance of the plunger rod 55 distal movement and to avoid hindrance of outward deflection of the trigger fingers 621, i.e. to avoid hindrance of activation of the trigger fingers 621 by the plunger rod 55 at the end of injection.

The proximal stop 22 may be arranged at the proximal end 204 of the outer body 2 and/or be defined at a proximal side of the finger flange 21, for axially abutting against the injection device 5. For instance, the proximal stop 22 of the outer body 2 axially abuts against a finger flange 21 of the injection device 5. The proximal stop 22 allows for preventing distal movement of the injection device 5 with respect to the casing 1 of the invention. Taking in account the axial length of the injection needle 53, the relative axial position of the proximal stop 22 and of the distal end 202 of the outer body 2 defines a predetermined needle exposure length. In other words, knowing the axial length of the injection needle 53, the axial length separating the proximal stop 22 from the distal end 202 of the outer body 2 defines the predetermined needle exposure length 531. Since the needle exposure length is physically determined by the outer body 2, the user can reliably prick at the right injection depth, and therefore at the right tissues, without requiring high skills. The outer body 2 may also include orthoradial abutments 23, Figure 5A, for rotationally securing the injection device 5 with respect to the casing 1, so that the injection device 5 cannot rotate within the casing 1. The orthoradial abutments 23 thus orthoradially abut against the injection device 5 when the injection is positioned within the casing 1.

With reference to Figure 3, the axial length separating the proximal stop 22 from the distal end 202 of the outer body 2 may be configured such that at least a portion of the needle shield 54 distally extends beyond the distal end 202 of the body 2. Therefore, the user can easily grasp and remove the needle shield 54.

With reference to Figures 5B-5C, the body 2 may include two slots 31 respectively extending on both sides of the snap-fit member 3. This provides a higher lever arm effect so that the clipping or unclipping of the injection device 5 with respect to the body 2 is made easier. The proximal end 301 of the snap-fit member 3 may be proximal to the proximal stop 22 of the body 2. The proximal stop 22 of the body 2 may be axially located between the proximal end 301 of the snap-fit member 3 and the inward radial protrusion 32 of the snap-fit member 3.

As visible in Figure 5A, the body 2 may include one or more radial bumps 24 configured for radially abutting against the injection device 5 when the injection device 5 is positioned within the body 2. This eliminates radial clearances between the injection device 5 and the body 2. The positioning of the injection device 5 is accordingly improved. The one or more bumps 24 may be configured for radially abutting against the flange 551 of the injection device 5. The one or more bumps 24 are axially located between the proximal stop 22 of the body 2 and the inward radial protrusion 32 of the snap-fit member 3. For instance, the body 2 includes two pairs of diametrically opposite bumps 24. The bumps 24 may be arranged on either side of the small diameter portion 251 of the proximal opening 25 of the body 2.

As illustrated in Figures 3 and 4A-4C, the distal end 202 of the body 2 may include a ring 206 which radially outwardly protrudes from the distal end 202 of the body 2. This increases the contact surface between the distal end 202 of the body 2 and the injection site. A distal side of the ring 206 delimits a distal abutment surface 207 configured for axially abutting against the injection site. The ring 206 may extend all around the distal end 202 of the body 2. In another embodiment (not shown), the ring 206 may extend only partially around the distal end 202 of the body 2.

The body 2 may include two diametrically opposite snap-fit members 3. The body 2 defines a proximal opening 25 for receiving the injection device 5, more specifically, a flange 551 of the injection device 5. The proximal opening 25 of the body 2 and the flange 551 of the injection device may be complementarily shaped. The proximal opening 25 may have an oblong shape. The proximal opening 25 has a large diameter portion 252 and a small diameter portion 251 which is lower than the large diameter portion. The snap-fit members 3 may be diametrically opposite with respect to the large diameter portion 252 of the proximal opening 25 of the body 2.

The one or more snap-fit members 3 are configured for axially facing the proximal stop 22 so that the injection device 5 is axially blocked within the body 2 between the snap-fit member 3 and the proximal stop 22. The snap-fit member 3 may be supported by the finger flange 21 of the body 2. The proximal stop 22 may also be provided on the finger flange 21 of the body 2.

The snap-fit members 3 are radially outwardly deformable with respect to the outer body 2 between an unlocked position, Figure 5A, in which the snap-fit members 3 allow for axial insertion or removal of the injection device 5, and a locked position, Figure 6A, in which the snap-fit members 3 axially block the injection device 5 within the outer body 2. That is, the injection device 5 is distally blocked by the proximal stop 22 of the outer body 2 and one or more snap-fit members 3. The snap-fit members 3 may be configured for axially abutting against a proximal side of the finger flange 21 of the injection device 5.

The release position of the snap-fit member 3 may be a deformed position. The locked position of the snap-fit member 3 may be, or may not be, a rest position of the snap-fit member 3. Specifically, the locked position may be a slightly deformed position of the snap-fit member 3. At any rate, the locked position may be a less deformed position than the release position of the snap-fit member 3. The rest position may be radially inwardly located with respect to the locked position. Thus, the snap-fit member 3 may exert a permanent pressure against the injection device 5 when this injection device 5 is located inside the body 2. The locked position is radially inwardly located with respect to the release position. Thus, the snap-fit member 3 radially outwardly deforms to move from the locked position to the release position. The rest position is when the snap-fit member 3 is submitted to no effort, and no injection device 5 is present in the casing 1.

With reference to Figure 6B, the one or more snap-fit members 3 include an inward radial protrusion 32 having an inclined distal side 321 for abutting against the injection device. This distal side 321, inclined with respect to the longitudinal axis A and with respect to a transversal plane orthogonal to this longitudinal axis A, permits to avoid any gap between the snap-fit member 3 and the injection device 5 when the snap-fit member 3 is in the locked position. This avoids wobbling of the injection device 5. The distal side 321 of the inward radial protrusion 32 is oriented, i.e. faces, towards the central longitudinal axis A and towards a distal 202 end of the body 2. The inward radial protrusion 32 may include an opposite proximal side 322, which may be inclined with respect to the longitudinal axis A, to ease deformation of the snap-fit member 3 and thus passage of the injection device 5 over the inward radial protrusion 32 when the injection device 5 is inserted within the body 2. The proximal side 322 of the inward radial protrusion 32 is oriented, i.e. faces, towards the central longitudinal axis A and the proximal direction. The proximal side 322 of the inward radial protrusion 32 may be axially longer than the distal side 321 of the inward radial protrusion 32. The slope of the proximal side 322 of the inward radial protrusion 32 is thus less steep than the slope of the distal side 321 with respect to the longitudinal axis A.

Still with reference to Figure 6B, the snap-fit members 3 may be proximally extending legs having a proximal end 301 fixed to the body, a free distal end 302, a bent portion 33 arranged between the proximal end 301 and the free distal end 302, and a pushing surface 34 defined between the bent portion 33 and the free distal end 302 for allowing a user to outwardly deform the snap-fit member 3 from the locked position to the release position. The inward radial protrusion 32 may be located between the bent portion 33 and the proximal end 301. The pushing surface 34 is an inner surface of the snap-fit member 3, i.e. faces the central longitudinal axis A. The snap-fit members 3 may include a (first) straight portion 351 extending between the proximal end 301 and the bent portion 33. The first straight portion 351 may be parallel to the central longitudinal axis in the rest and/or the locked position. The snap-fit member 3 may include a (second) straight portion 352 extending between the bent portion 33 and the free distal end 302. The second straight portion 352 is oblique. Specifically, the second straight portion 352 is inclined with respect to the central longitudinal axis A, such that the second straight portion 352 extends radially outwardly from the bent portion 33 towards the free distal end 302. The free distal end 302 is furthest from the central longitudinal axis A than the proximal end 301. The free distal end 302, or more specifically the second straight portion 352, may proximally protrude from the body 2, or more specifically from the finger flange 21 of the body 2. Therefore, the free distal end 302, the second straight portion 352, and thus the pushing surface 34 can be easily accessible to the user. Removal of the injection device 5 from the body 2 is made easier.

The inward radial protrusion 32, the first portion 351 and/or the second portion 352 may be made of a single piece. The snap-fit member 3 may thus be made of a single piece. Also, the outer body 2, the proximal stop 22, the finger flange 21, and/or the snap-fit members 3 may be made of a single piece.

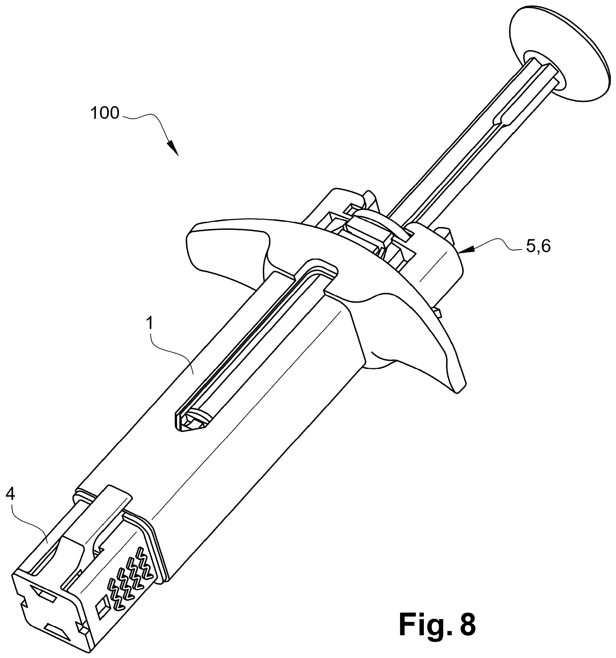

As illustrated in Figure 8, the casing may include a cap 4 removably attached to the distal end 202 of the body for protecting the needle shield 54 and the injection device 5 before use. The cap 4 includes a gripping member 43 configured for gripping the needle shield 54 of the injection device 5, such that removal of the cap 4 from the body 2 entails removal of the needle shield 54 from the injection device 5, and a release element 44 configured for releasing the needle shield 54. The cap 4 is configured for being removed from the outer body 2 by pulling the cap 4 in the distal direction.

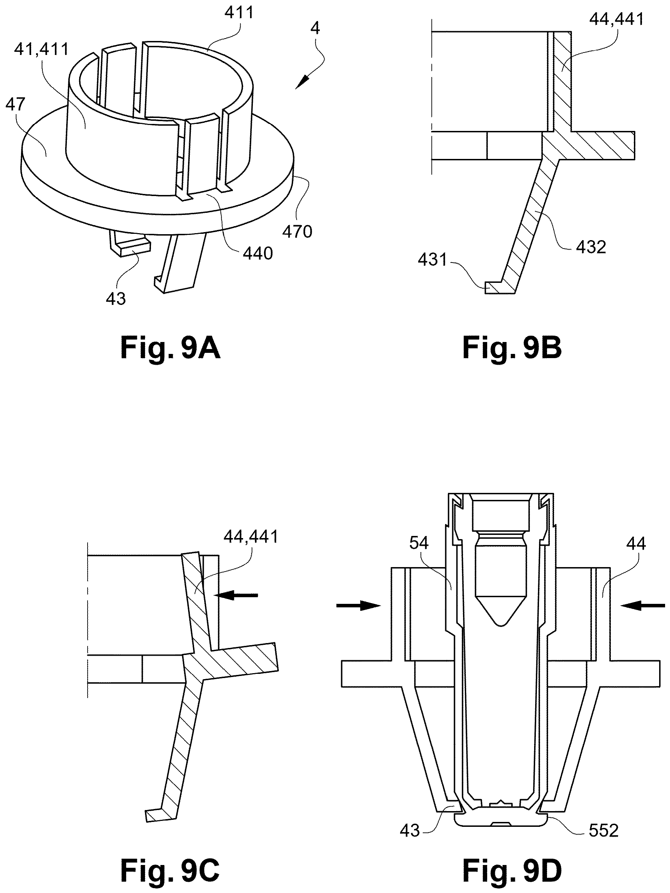

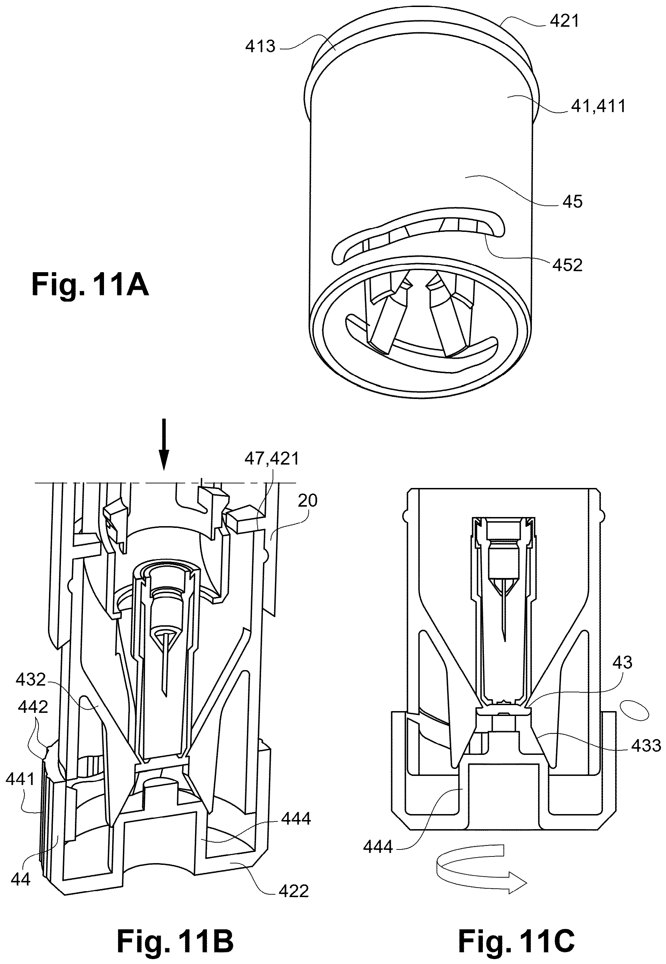

The cap 4 includes one or more attachment portions 41, which may be arranged at a proximal end 421 thereof, for removably attaching the cap 4 to the distal end 202 of the outer body 2. The attachment portions 41 may allow for instance a snap-fit or a friction-fit connection between the cap 4 and the outer body 2. In the embodiment illustrated in Figure 9A, the attachment portions 41 may be in the form of two hemicylindrical sidewalls 411 configured for frictionnally engaging an inner surface of the lateral wall 20 of the outer body 2. In the embodiment illustrated in Figure 9A, 10A, 13A or 11A-12B, the attachment portion 41 is a square or cylindrically shaped sidewall 411 configured for frictionnally engaging an inner surface of the lateral wall 20 of the outer body 2. Any other shape capable of frictionnally engage the inner surface of the lateral wall 20 of the outer body 2 may be appropriate. The attachment portion 41 may further include one or more axial ribs 412 outwardly protruding from the sidewall 411 for increasing friction between the outer body 2 and the cap 4. In another embodiment illustrated in Figures 11A-12B, the attachment portion 41 includes a cylindrical sidewall 411, which may be arranged at the proximal end 421 of the cap 4, for frictionnally engaging an inner surface of the lateral wall 20 of the outer body 2. The attachment portions 41 may further include a circumferential rib (or groove) 413 for complementarily engaging a circumferential groove (or rib) of the outer body 2.

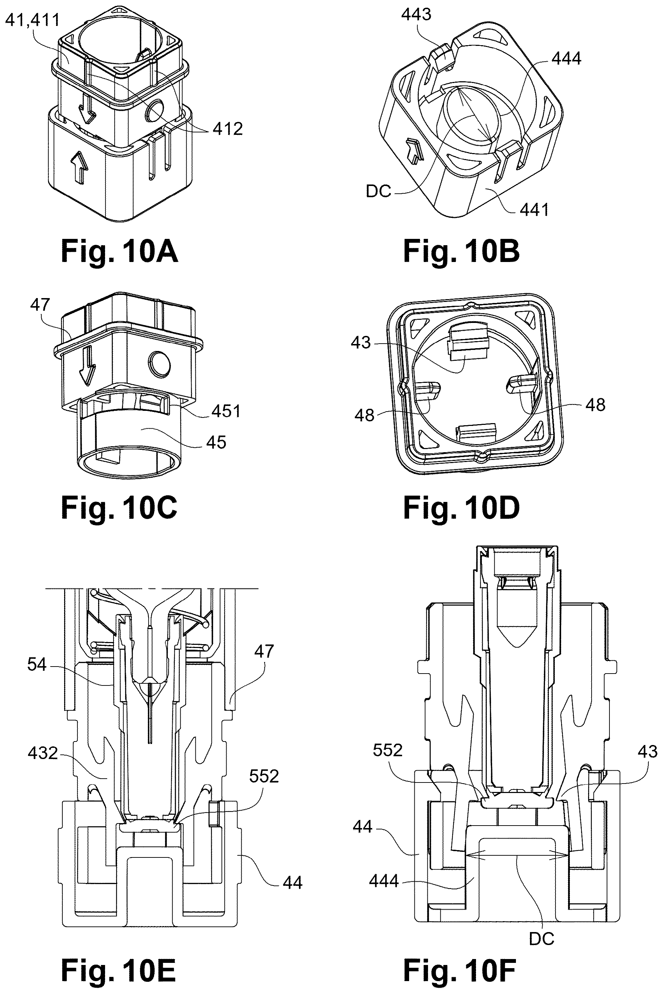

The cap 4 further includes one or more gripping members 43 configured for engaging the needle shield 54 such that removal of the cap 4 from the outer body 2 entails removal of the needle shield 54 from the medical container 51 of the injection device 5. The gripping member 43 may engage a lateral wall, a proximal end, or a distal end of the needle shield 54. For instance, the gripping member 43 may engage an axial gap (not shown) between a proximal end of the needle shield 54 and a distal shoulder of the barrel 52 of the medical container 51, or may engage a recess 552, see for instance Figure 10E, arranged at the distal end of the needle shield 54.

With reference to Figures 9A-9D, the gripping member 43 includes an inward radial tab or hook 431 arranged at a distal end of a radially movable leg 432 for engaging the recess 552 at the distal end of the needle shield 54. The cap 4 may include two diametrically oppposite gripping members 43. The gripping members 43 are radially movable between between a gripping position, in which they grip the needle shield 54, and a release position, outwardly radially located with respect to the gripping position, in which they release the needle shield 54 so that the needle shield 54 can be ejected. Movement of the gripping members 43 is caused by one or more release elements 44 activated by the user. The release elements 44 may be axial levers. The axial levers may be proximally protruding at a inner end of a radial arm 440, while the gripping members 43 may be distally protruding at the inner end of the radial arm 440. The radial arm 440 radially inwardly extends from an outer flange 470 which delimits a proximal stop 47 whose function will be described below. The radial arm 440 forms a resiliently deformable connecting member for connecting the gripping member 43 to the rest of the cap 4. The release elements 44 have a handling feature 441, such as an outer pressing surface. The release elements 44 are radially movable between a first (initial) position, Figure 9B, in which they are not pressed by the user, and an ejection position, Figure 9C, radially inwardly located with respect to the first position, in which they are pressed by the user to allow for ejection of the needle shield 54 outside the cap 4. Movement of the release elements 44 from the first to the ejection position is caused by the user radially inwardly pressing against the outer pressing surfaces 441. Movement to the first position may be caused by the resiliently deformable protrusion returning back towards its rest state. Radial inward movement of the release elements 44 to the ejection position here entails radial outward movement of the gripping members 43 to their release position.

With reference to Figures 10A-10F, the gripping member 43 includes an inward radial tab or hook 431 arranged at an intermediate portion of a radially movable leg 432 for engaging the recess 552 at the distal end of the needle shield 54. The cap 4 may include two diametrically oppposite gripping members 43. The gripping members 43 are radially movable between between a gripping position, Figure 10E, in which they grip the needle shield 54, and a release position, Figure 10F, outwardly radially located with respect to the gripping position, in which they release the needle shield 54 so that the needle shield 54 can be ejected. Movement of the gripping members 43 is caused by one or more release elements 44 activated by the user. The release element 44 may include a cam 444 proximally protruding from a distal end 422 of the cap. The cam 444 is arranged between the distal ends of the two diametrically opposite gripping members 43. The cam 444 may have a non-circular shape, such as an oblong shape.

Stil with reference to Figures 10A-10F, the release element 44 has a handling feature 441, such an outer surface configured for being handled by the user. The release element may have a non-circular shape, such as a square or rectangular shape. The release elements 44 are rotationally movable between a first position, Figure 10E, in which the cam 444 lets the gripping members 43 in their gripping position, and an ejection position, Figure 10F, at 90° from the first position, in which a larger diameter DC of the cam 444 extends between the gripping members 43 such that the gripping members 43 are radially outwardly moved away from each other, thereby reaching their release position. Movement of the release element 44 from the first to the ejection position is caused by the user rotating the release element 44 around the longitudinal axis A with respect to the attachment portion 41 of the cap. Movement of the release element 44 from the ejection to the first position may be either caused by the user rotating the release element 44 back around the longitudinal axis A with respect to the attachment portion 41 of the cap, so that the release element 44 has two stable positions, or by a torsion spring (not shown), so that the release element 44 is monostable, the single stable position being the first position. Rotation of the release element 44 may be guided by a guiding portion 45 of the cap, which may have a cylindrical shape complementary to the shape of an inner cavity of the release element 44. The guiding portion 45 may include one or more circumferential slots 451 for accommodating a hook of a snap-fit member 443 of the release element 44, the snap-fit member 443 axially securing the release element 44 with respect to the guiding portion 45 of the cap.

With reference to Figures 11A-11C, the gripping member 43 includes an inward radial tab or hook arranged at an intermediate portion of a radially movable leg 432 for engaging the recess 552 at the distal end of the needle shield 54. The cap 4 may here include two pairs of diametrically oppposite gripping members 43. The gripping members 43 are radially movable between between a gripping position, Figure 11B, in which they grip the needle shield 54, and a release position, outwardly radially located with respect to the gripping position, in which they release the needle shield 54 so that the needle shield 54 can be ejected. Movement of the gripping members 43 is caused by one or more release elements 44 activated by the user. The release element 44 may include a cam 444 proximally protruding from a distal end 422 of the cap. The cam 444 is arranged between the distal ends 434 of the two diametrically opposite gripping members 43. The cam 444 may have a cylindrical shape.

Still with reference to Figures 11A-11C, the release element 44 has a handling feature 441, such an outer surface configured for being handled by the user, this outer surface including axial handling ribs 442 for easing rotation of the release element 44 with respect to the outer body 2. The release element 44 may have a cylindrical shape. The release element 44 is rotationally and axially movable between a first position, in which the cam 444 lets the gripping members 43 in their gripping position, and an ejection position, at 90° from the first position and proximal to the first position, in which the cam 444 abuts against ramp surfaces 433 provided at the distal end of the gripping members 43 such that the gripping members 43 are radially outwardly moved away from each other, thereby reaching their release position. Movement of the release element 44 from the first to the ejection position is caused by the user rotating the release element 44 around the longitudinal axis A with respect to the attachment portion 41 of the cap 4. Movement of the release element 44 from the ejection to the first position may be either caused by the user rotating the release element 44 back around the longitudinal axis A with respect to the attachment portion 41 of the cap, so that the release element 44 has two stable positions, or by a torsion spring (not shown), so that the release element 44 is monostable, the single stable position being the first position. Rotation of the release element 44 may be guided by a guiding portion 45 of the cap, which may have a cylindrical shape complementary to the shape of an inner cavity of the release element 44. The guiding portion 45 may include one or more helical slots 452 for accommodating a hook of a snap-fit member of the release element 44, such that the movement of the release element 44 with respect to the cap 4 is simultaneously axial and rotational.

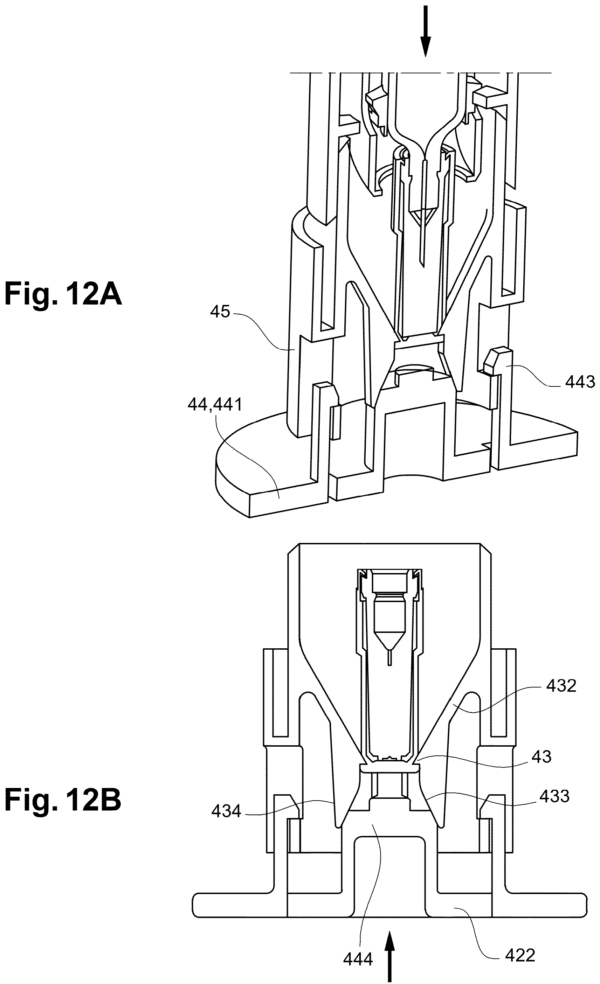

Turning now to Figures 12A-12B, the gripping member 43 includes an inward radial tab or hook 431 arranged at an intermediate portion of a radially movable leg 432 for engaging the recess 552 at the distal end of the needle shield 54. The cap 4 may include two pairs of diametrically oppposite gripping members 43. The gripping members 43 are radially movable between between a gripping position, Figure 12B, in which they grip the needle shield 54, and a release position, outwardly radially located with respect to the gripping position, in which they release the needle shield 54 so that the needle shield 54 can be ejected. Movement of the gripping members 43 is caused by one or more release elements 44 activated by the user. The release element 44 may include a cam 444 proximally protruding from a distal end 422 of the cap. The cam 444 is arranged between the distal ends 434 of the two diametrically opposite gripping members 43. The cam 444 may have a cylindrical shape.

The release element 44 has a handling feature 441, such an outer flange and/or a distal pushing surface to allow handling of the release element 44 by the user. The release element 44 here is axially movable between a first position, in which the cam 444 lets the gripping members 43 in their gripping position, and an ejection position, proximal to the first position, in which the cam 444 abuts against ramp surfaces 433 provided at the distal end 434 of the gripping members 43 such that the gripping members 43 are radially outwardly moved away from each other, thereby reaching their release position. Movement of the release element 44 from the first to the ejection position is caused by the user moving the release element 44 in the proximal direction along the longitudinal axis A. Movement of the release element 44 from the ejection to the first position may be caused by the user. Axial movement of the release element 44 may be guided by a guiding portion 45 of the cap, which may include one or more windows for accommodating a hook of a snap-fit member 443 of the release element 44.

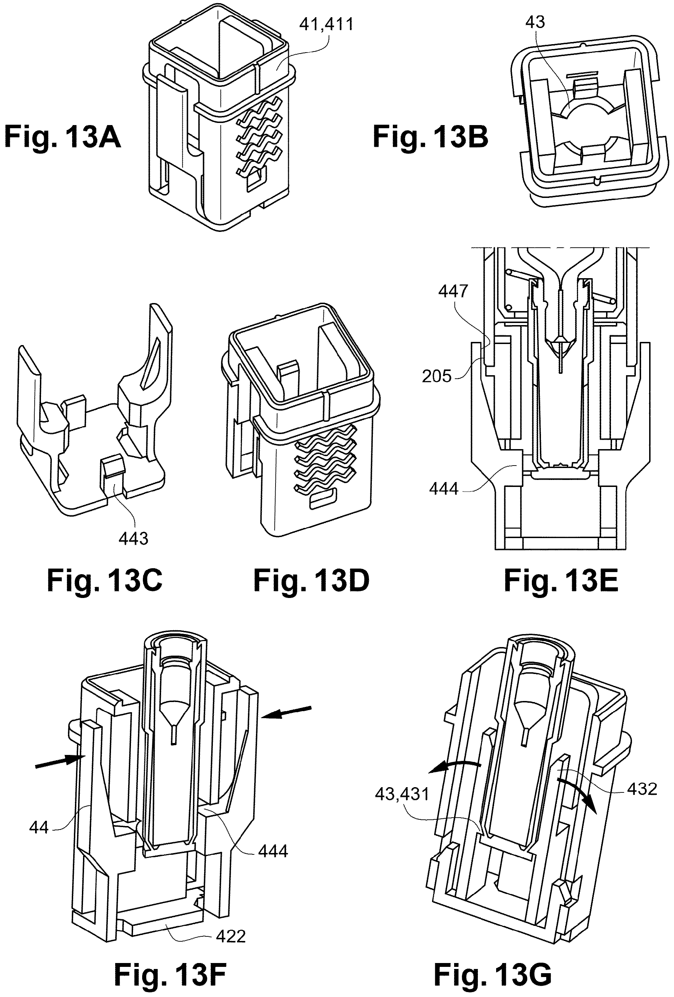

With reference to Figures 13A-13G, the gripping member 43 includes an inward radial tab or hook 431 arranged at an intermediate portion of a radially movable leg 432 for engaging the recess 552 at the distal end of the needle shield 54. The cap 4 may include two diametrically oppposite gripping members 43. The gripping members 43 are radially movable between between a gripping position, in which they grip the needle shield 54, and a release position, outwardly radially located with respect to the gripping position, in which they release the needle shield 54 so that the needle shield 54 can be ejected. Movement of the gripping members 43 is caused by one or more release elements 44 activated by the user. The release element 44 may include one or more radial cams 444 provided at two proximally protruding legs axially protruding from a distal end 422 of the cap 4. The cams 444 are configured to extend between the proximal ends of the two gripping members 43, i.e. proximal to the inward radial tabs.

The release element 44 here is rotationally and axially fixed with respect to the attachment portion 41 of the cap, and may be attached to the rest of the cap 4 by one or more snap-fit members 443. The proximally protruding legs of the release element 44 are radially movable between a first position, Figure 13E, in which their cams 444 let the gripping members 43 in their gripping position, and an ejection position, radially inwardly located with respect to the first position, in which the cam 444 abuts against the gripping members 43 such that the gripping members 43 are radially outwardly moved away from each other, thereby reaching their release position. Movement of the proximally protruding legs from the first to the ejection position is caused by the user radially inwardly pushing against an outer surface of these proximally protruding legs. Movement of the release element 44 from the ejection to the first position may be due to the resiliency of the proximally protruding legs.

In order to prevent release of the needle shield during removal of the cap from the body, the release element 44 may include a radial blocking surface 447, Figure 13E, which may be arranged at an inner side of the proximal end of the proximally protruding legs for radially abutting against a corresponding blocking surface 205 of the outer body 2, this blocking surface 205 of the outer body 2 being defined by the lateral wall 20 of the outer body 2 at a distal end thereof. Thus, as long as the cap 4 is attached to the outer body 2, the blocking surfaces 205, 447 prevent movement of the release element 44 to the ejection position.

The cap 4 may include a proximal stop 47 configured for axially abutting against the outer body 2 so that attachment of the cap 4 to the outer body 2 is stopped at a predetermined axial position. This enables to make sure that the gripping member 43 of the cap 4 does grip the needle shield 54 of the injection device 5. In the embodiment illustrated in Figure 9A, the proximal stop 47 is a proximal side of the outer flange 470 arranged between the attachment portion 41 and the gripping member. As shown in Figure 10C, the proximal stop 47 may be a peripheral protrusion or rib outwardly protruding from the sidewall of the cap 4. In the embodiment illustrated in Figure 11B, the proximal stop 47 is the proximal end 421 of the cap.

In some embodiments, the cap 4 may include centering features 48 for centering and guiding the needle shield 54 with respect to the cap, so that the needle shield 54 properly engages the gripping member 43 of the cap. As illustrated in Figure 10D, the centering features 48 may include one or more, for instance two diametrically opposite axial ribs protruding from a lateral wall of the cap 4. The axial ribs are arranged at 90° with respect to the two diametrically opposite gripping members 43.

It is contemplated that the cap 4 may be made of a single piece, as illustrated for instance in Figures 9A-9D, in which the release element 44, the gripping member, and the attachment portion 41 form a single part. In other embodiments, the cap 4 may be made of two or more assembled distinct parts. For instance, as illustrated in Figures 10A-10F, 11A-11C, 12A-12B, and 13A-13G, the release element 44 and the attachment portion 41 of the cap 4 are two distinct parts of the cap. In most embodiments, the gripping member 43 and the attachment portion 41 of the cap 4 may be made of a single piece.

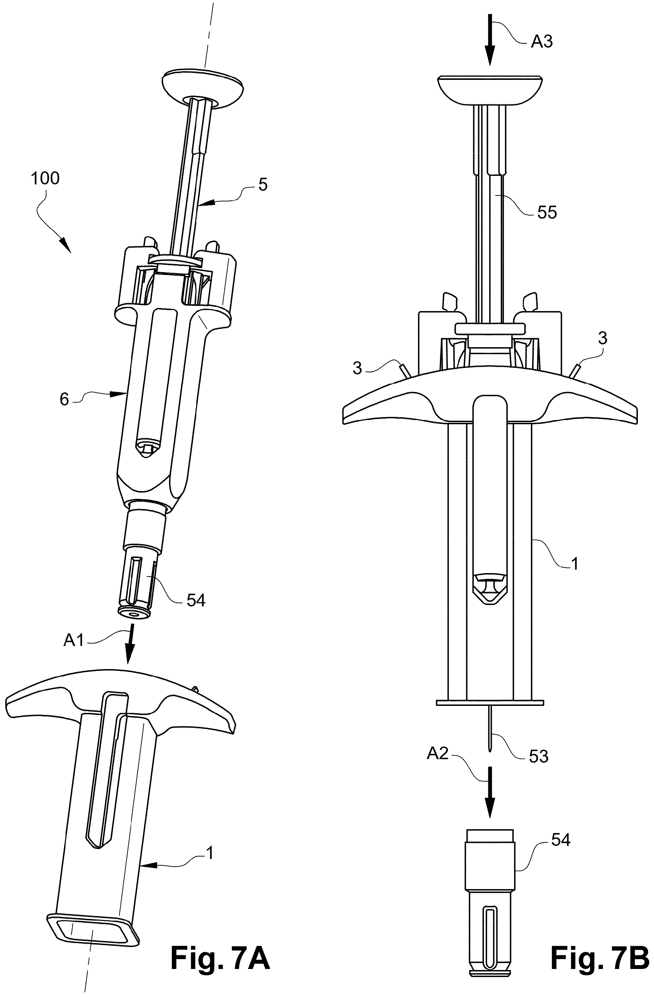

Operation of the casing 1 according will now be described with reference to Figures 7A-7E.

The user first inserts the injection device 5 within the casing 1, as illustrated by arrow A1 in Figure 7A. If there is a cap 4, the needle shield 54 of the injection device 5 gets engaged with the gripping member 43 of the cap 4 during insertion. The finger flange 551 of the safety device 6 or of the injection device 5 passes over the inward radial protrusion 32 of the snap-fit members 3. The snap-fit members 3 radially outwardly deflect to the release position. After passage of the injection device 5 over the inward radial protrusion 32 of the snap-fit members 3, the snap-fit members 3 automatically go back towards their initial position, i.e. move radially inwardly, and the injection device 5 comes into abutment against the proximal stop 22 of the casing 1. As a result, the injection device 5 gets axially trapped between the inward radial protrusion 32 of the snap-fit members 32 and the proximal stop 22 of the body 2. The injection device 5 is secured within the casing 1.

If there is a cap 4, the user then removes the cap 4 by distally pulling the cap 5 off the casing 1. Due to the gripping member 43 engaged with the needle shield 54, the needle shield 54 is removed too. If there is no cap 4, the user directly grasps the needle shield 54 and removes the needle shield 54, arrow A2 in Figure 7B. Anyway, the injection needle 53 is unveiled. The user can then perform injection.

The distal end 202 of the outer body 2 is positioned in abutment against the injection site, which ensures to prick at a predetermined injection depth. The user pushes the plunger rod 55 in the distal direction, arrow A3 in Figure 7B, so that the medical product contained within the injection device 5 is expelled towards the injection site via the injection needle 53. At the end of injection, the plunger rod 55 moves the trigger fingers 621 apart, thereby triggering deployment of the needle guard 62 which surrounds the injection needle 53 to protect the user against needle stick injuries.

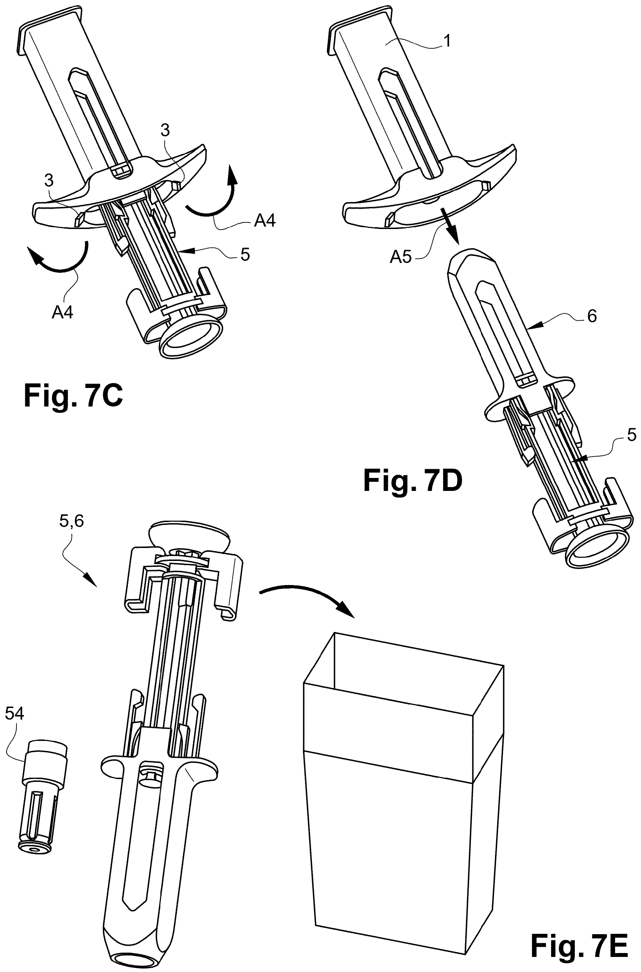

The user deflects the snap-fit members 3 radially outwardly to move them to their release position, see arrows A4 in Figure 7C, so that the used injection device 5 can be withdrawn from the casing 1, by gravity or pulled by the user, arrow A5 in Figure 7D. If there was a cap 4, the user can move the release element 44 of the cap 4 to its ejection position, such that the gripping member 43 of the cap 4 releases the needle shield 54. The used injection device 5 and used needle shield 54 can then be discarded in an appropriate waste container, Figure 7E. If there was a cap 4, the user may re-attach the cap 4 to the outer body 2 of the casing 1 so that the casing 1 can be re-used with another injection device 5.

It is readily understandable from the above description that the casing 1 of the invention can thus be re-used instead of being discarded. This improves sustainability. Besides, the casing 1 allows for a better handling by the user and enables to control the needle exposure length and by extension the injection depth without requiring a high accuracy from the user. The casing 1 is therefore easier to use. It is to be understood that the present invention is not limited to the embodiments described above and illustrated in the drawings; rather, the skilled person will recognize that many changes and modifications may be made within the scope of the appended claims.

## Claims

1. Casing (1) configured for receiving an injection device (5), the casing (1) including:
a body (2) delimiting an inner cavity for receiving the injection device (5), the body (2) including a proximal end (204) and a distal end (202) configured for axially abutting against an injection site,
a proximal stop (22) arranged on the body (2) for blocking distal movement of the injection device (5) at a predetermined axial position within the body (2),
a snap-fit member (3) arranged on the body (2) for blocking proximal movement of the injection device (5), the snap-fit member (3) being resiliently deformable between a release position allowing insertion or removal of the injection device (5), and a locked position in which the snap-fit member (3) prevents removal of the injection device (5) from the body (2).

2. Casing (1) according to the preceding claim, wherein the body (2) includes two diametrically opposite snap-fit members (3).

3. Casing (1) according to any one of the preceding claims, wherein the snap-fit member (3) axially faces the proximal stop (22) so that the injection device (5) is axially blocked within the body (2) between the snap-fit member (3) and the proximal stop (22).

4. Casing (1) according to any one of the preceding claims, wherein the snap-fit member (3) and the proximal stop (22) are provided on a finger flange (21) of the body (2).

5. Casing (1) according to any one of the preceding claims, wherein the snap-fit member (3) and the body (2) are made of a single piece.

6. Casing (1) according to any one of the preceding claims, wherein the snap-fit member (3) includes an inward radial protrusion (32) having an inclined distal side (321) for abutting against the injection device (5).

7. Casing (1) according to any one of the preceding claims, wherein the release position of the snap-fit member (3) is a deformed position.

8. Casing (1) according to any one of the preceding claims, wherein the snap-fit member (3) is a proximally extending leg having a proximal end (301) fixed to the body (2), a free distal end (302), a bent portion (33) arranged between the proximal end (301) and the free distal end (302), and a pushing surface (34) defined between the bent portion (33) and the free distal end (302) for allowing a user to outwardly deform the snap-fit member (3) from the locked position to the release position.

9. Casing (1) according to any one of the preceding claims, wherein the body (2) includes two slots (31) respectively extending on each side of the snap-fit member (3).

10. Casing (1) according to any one of the preceding claims, wherein the body (2) includes one or more radial bumps (24) configured for radially abutting against the injection device (5) when the injection device (5) is positioned within the body (2).

11. Casing (1) according to any one of the preceding claims, wherein the body (2) includes a see-through window (201).

12. Casing (1) according to any one of the preceding claims, wherein the distal end (202) of the body (2) includes a ring (206) which radially outwardly protrudes from the distal end (202) of the body (2).

13. Casing (1) according to any one of the preceding claims, wherein the casing (1) includes a cap (4) removably attached to the body (2), the cap (4) including a gripping member (43) configured for gripping a needle shield (54) of the injection device (5), such that removal of the cap (4) from the body (2) entails removal of the needle shield (54) from the injection device (5), and a release element (44) configured for releasing the needle shield (54).

14. Drug delivery device (100) including a casing (1) according to any one the preceding claims, and an injection device (5) arranged within the casing (1).
